# EUROPEAN PATENT APPLICATION

(11) **EP 2 987 471 A2**
(43) Date of publication of application: **24.02.2016**
(21) Application number: 14193649.2
(22) Date of filing: 18.11.2014
(51) Int. Cl.: A61F 5/01

(54) **Rehabilitation footwear**

(30) Priority: 18.08.2014 TW 103128358
(71) Applicant: E-Life International Co., Ltd., New Taipei City (TW)
(72) Inventor: Lee, Shih-Hsiang, New Taipei City (TW); Chang, Yun-Teng, New Taipei City (TW)
(74) Representative: Becker Kurig Straus

(57) **Abstract**

A full-cover rehabilitation footwear comprises the shoe body (10), the lower back guard pad (20), the upper back guard pad (30), the lower front guard pad (50), the upper front guard pad (60) and the multiple fasteners (11, 12, 13, 14, 15, 16, 17, 18). A supporting-type rehabilitation footwear comprises the shoe body (10), the lower back guard pad (20), the middle back guard pad (40), the lower front guard pad (50), the middle front guard pad (70) and the multiple fasteners (11, 12, 13, 14, 15, 16). A frame-type rehabilitation footwear comprises the shoe body (10), the two supporting frames (100, 110) and the multiple fasteners (15, 16, 17, 18). An adjustable rehabilitation footwear is provided.

## Description

### 1. Field of the Invention

The present invention relates to a rehabilitation footwear, especially to a rehabilitation footwear that can fix bones of an injured foot and has adjustable functions according to different stages of recovery and rehabilitation.

### 2. Description of the Prior Art

An injured person's foot after surgery, fracture, sprain or contusion needs to be fixed with plaster to fix soft tissues as well as bones in a right position. The purpose of using plaster is to prevent the soft tissues and the bones from shifting and causing a secondary injury and to enhance rehabilitation of the foot. To enhance rehabilitation of the soft tissues and the bones of the injured person, a rehabilitation footwear is available on the market. Conventional rehabilitation footwear includes an ankle rehabilitation footwear and a rehabilitation footwear. The ankle rehabilitation footwear is used for protecting a sole and an ankle of the foot. The high leg rehabilitation footwear is used for protecting a shank of the leg. The ankle rehabilitation footwear is restricted to rehabilitate an injured region of the sole and ankle of the foot. On the other hand, the high leg rehabilitation footwear is designed with an adjustable or nonadjustable height. The high leg rehabilitation footwear with adjustable height can be adjusted according to the injured region of the foot. However, the high leg rehabilitation footwear with adjustable height lacks a design for full cover protection and for different recovery stages of the foot. In other words, the conventional rehabilitation footwear lacks the adjustable function based on the recovery stages of the foot to enhance the recovery rate of the soft tissues as well as the bones of the injured person.

To overcome the shortcomings, the present invention provides a rehabilitation footwear to mitigate or obviate the aforementioned problems.

The main objective of the invention is to provide a rehabilitation footwear with an adjustable function according to the recovery stages of an injured person's foot.

The rehabilitation footwear in accordance with the present invention has a shoe body, multiple fasteners, a back guard pad, a front guard pad, two supporting frames and an encircling band.

The shoe body comprises multiple buckles and two concave grooves.

Each of the multiple fasteners comprises a buckle ring and a band ring. The buckle ring is fastened with one of the multiple buckles of the shoe body.

The back guard pad comprises a lower back guard pad, a middle back guard pad, and an upper back guard pad. The lower back guard pad comprises two protrusion parts, multiple holes and multiple perforations. The two protrusion parts of the back guard pad are interlocked with the two concave grooves of the shoe body. The middle back guard pad comprises multiple buckles and multiple clasps. The multiple buckles of the middle back guard pass through the multiple holes of the lower back guard pad and are fastened with the buckle rings of the multiple fasteners. The multiple clasps of the middle back guard are clasped with the perforations of the lower back guard pad. The upper back guard pad comprises multiple buckles and multiple clasps. The multiple buckles of the upper back guard pad pass through the multiple holes of the lower back guard pad and are fastened with the buckle rings of the multiple fasteners. The multiple clasps of the upper back guard pad are clasped with the multiple perforations of the lower back guard pad.

The front guard pad comprises a lower front guard pad, a middle front guard pad, and an upper front guard pad. The lower front guard pad comprises a clasp. The middle front guard pad comprises a perforation clasped with the clasp of the lower front guard pad. The upper front guard pad comprises a perforation clasped with the clasp of the lower front guard pad.

Each of the two supporting frames comprises a protrusion part and multiple band rings. The protrusion part of each of the two supporting frames is interlocked with each of the two concave grooves of the shoe body.

The encircling band passes through the multiple band rings of the two supporting frames that are positioned correspondingly to each other.

A full-cover rehabilitation footwear is built up by combination of the shoe body, the lower back guard pad, the upper back guard pad, the lower front guard pad and the upper front guard pad, and fixed with the injured person's foot by the encircling band passing through the band rings of the multiple fasteners corresponding to each other.

A supporting-type rehabilitation footwear is built up by combination of the shoe body, the lower back guard pad, the middle back guard pad, the lower front guard pad and the middle front guard pad, and fixed with the injured person's foot by the encircling band passing through the band rings of the multiple fasteners corresponding to each other.

A frame-type rehabilitation footwear is built up by combination of the shoe body and the two supporting frames, and fixed with the injured person's foot by the encircling band passing through the multiple band rings of the two supporting frames corresponding to each other.

### In the drawings

Fig. 1 is a perspective exploded view of a rehabilitation footwear in accordance with the present invention;
Fig. 2A is an enlarged view of a lower back guard pad of the rehabilitation footwear in Fig. 1;
Fig. 2B is an enlarged view of an upper back guard pad of the rehabilitation footwear in Fig. 1;
Fig. 3 is a side view of the rehabilitation footwear in Fig. 1;
Fig. 4 is a perspective view of a first embodiment of the rehabilitation footwear in accordance with the present invention;
Fig. 5 is a perspective view of a second embodiment of the rehabilitation footwear in accordance with the present invention; and
Fig. 6 is a perspective view of a third embodiment of the rehabilitation footwear in accordance with the present invention.

### The first embodiment

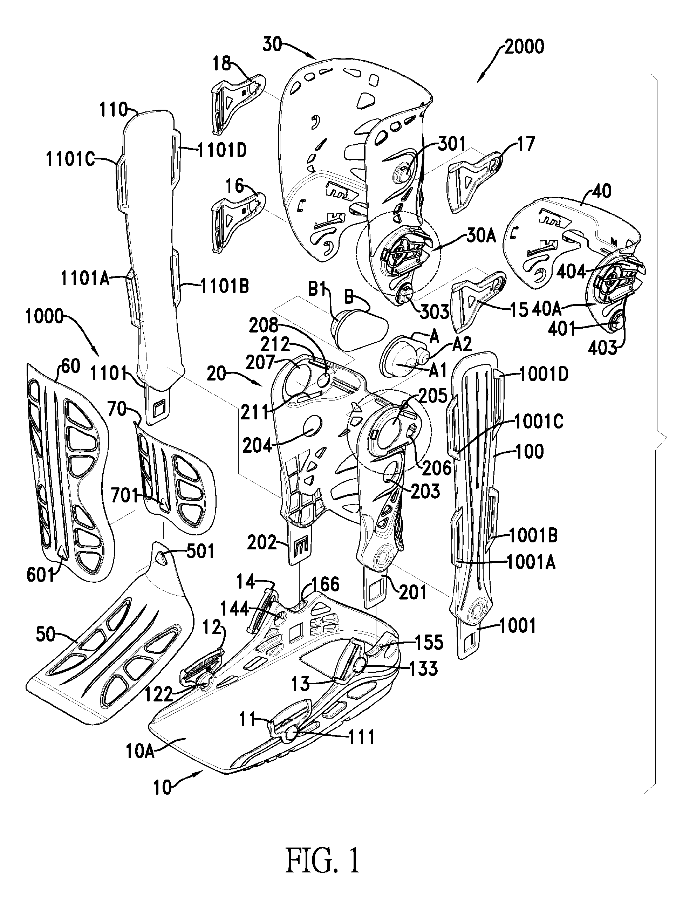

With reference to Fig. 1, a first embodiment of a rehabilitation footwear in accordance with the present invention comprises a shoe body 10, a back guard pad 1000, a front guard pad 2000, a first fastener 11, a second fastener 12, a third fastener 13, a fourth fastener 14, a fifth fastener 15, a sixth fastener 16, a seventh fastener 17, an eighth fastener 18, a first fill A and a second fill B.

The shoe body 10 comprises two lateral sides and a back end respectively corresponding to bilateral sides and a heel of a human's foot. The two lateral sides and the back end of the shoe body 10 form a capacity space for accommodating the human's foot. The two lateral sides comprise a first buckle 111, a second buckle 122, a third buckle 133, and a fourth buckle 144. The back end of the shoe body 10 comprises a first concave groove 155 and a second concave groove 166.

The back guard pad 1000 comprises a lower back guard pad 20 and an upper back guard pad 30. The lower back guard pad 20 comprises three lateral sides connected with each other at nearly right angle, allowing to be formed a lower capacity space for accommodating a back of a human's shank. The lower capacity space comprises a lower end and an upper end. Two of the lateral sides of the lower back guard pad 20 opposite each other and corresponding to the lower end of the lower capacity space extend and form a first protrusion part 201 and a second protrusion part 202. The first protrusion part 201 and the second protrusion part 202 are respectively interlocked with the first concave groove 155 and the second concave groove 166 of the shoe body10, allowing the back guard pad 1000 to be fixed with the shoe body 10. The two opposite lateral sides of the lower back guard pad 20 comprise a first hole 203 and a second hole 204. The two lateral sides of the lower back guard pad 20 opposite each other and corresponding to the upper end of the capacity space extend outwards and form a small capacity space.

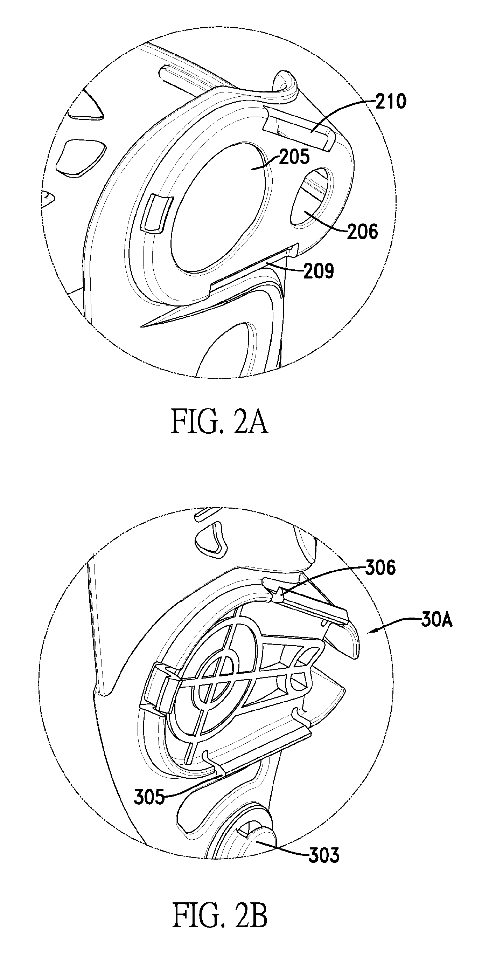

With reference to Fig. 1 and Fig. 2A, the small capacity space corresponding to the two lateral sides of the lower back guard pad 20 opposite each other forms a third hole 205, a fourth hole 206, a fifth hole 207, a sixth hole 208, a first perforation 209, a second perforation 210, a third perforation 211 and a fourth perforation 212.

The upper back guard pad 30 comprises three lateral sides connected with each other at nearly right angle, allowing to be formed an upper capacity space for accommodating the back of the human's shank. The upper capacity space comprises a lower end and an upper end. Two of the lateral sides of the upper back guard pad 30 opposite each other and corresponding to the upper end of the upper capacity space comprise a first upper buckle 301 and a second upper buckle. The two lateral sides of the upper back guard pad 30 opposite each other and corresponding to the lower end of the upper capacity space comprise a third upper buckle 303 and a fourth upper buckle. The third upper buckle 303 and the fourth upper buckle respectively pass through the first hole 203 and the second hole 204 of the lower back guard pad 20.

With reference to Fig. 1, Fig. 2A and Fig. 2B, the two lateral sides of the upper back guard pad 30 opposite each other and corresponding to the lower end of the upper capacity space extend outwards and form protrusion structures. One of the protrusion structures is a protrusion structure 30A. The protrusion structure 30A comprises a surrounding a recess, and the first fill A is placed in the recess of the protrusion structure 30A. The protrusion structure 30A is fitted into the small capacity space of the lower back guard pad 20. The other one of the protrusion structures is identical to the protrusion structure 30A and positioned oppositely to the protrusion structure 30A of the upper back guard pad 30. The other one of the protrusion structures comprises a surrounding wall having a recess, and the second fill B is placed in the recess of the other one of the protrusion structures. The protrusion structure 30A and the second fill B are fit into the small capacity space of the two opposite lateral sides of the lower back guard pad 20. The first fill A comprises a first bump A1 and a second bump A2. The second fill B comprises a third bump B1 and a fourth bump. The first bump A1 passes through the third hole 205 of the lower back guard pad 20, and the second bump A2 passes through the fourth hole 206 of the lower back guard pad 20. The third bump B1 passes through the fifth hole 207 of the lower back guard pad 20, and the fourth bump passes through the sixth hole 208 of the lower back guard pad 20.

Two lateral sides of the surrounding wall of the protrusion structure 30A comprise a first clasp 305 and a second clasp 306 formed toward outside of the recess. The first clasp 305 passes through and is clasped with the first perforation 209 of the lower back guard pad 20. The second clasp 306 passes through and is clasped with the second perforation 210 of the lower back guard pad 20, allowing the lower back guard pad 20 to be fixed with the upper back guard pad 30.

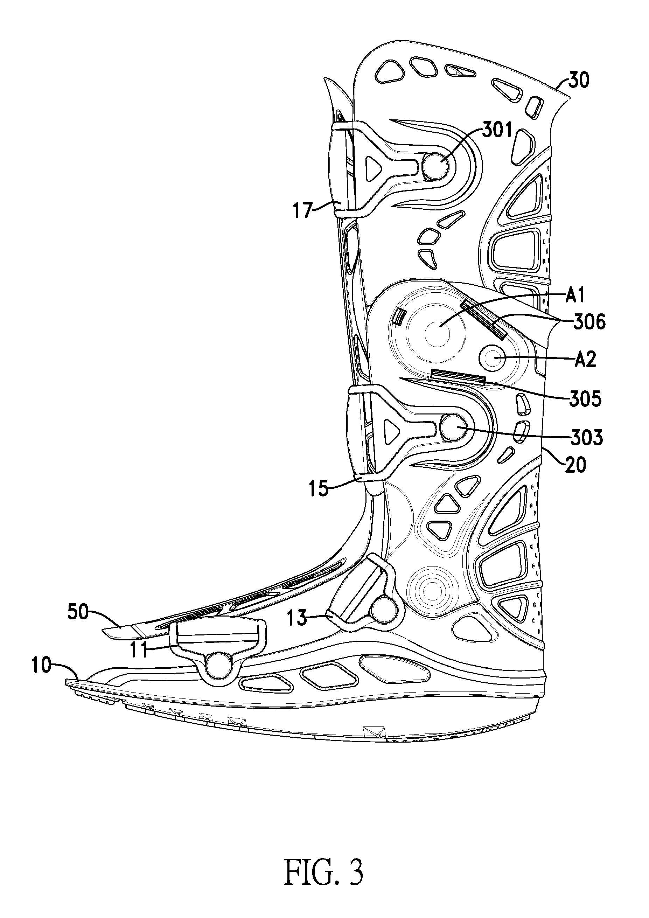

With reference to Fig. 1 and Fig. 3, the first fastener 11, the second fastener 12, the third fastener 13, the fourth fastener 14, the fifth fastener 15, the sixth fastener 16, the seventh fastener 17 and the eighth fastener 18 each comprises two ends. One end of each of the first fastener 11, the second fastener 12, the third fastener 13, the fourth fastener 14, the fifth fastener 15, the sixth fastener 16, the seventh fastener 17 and the eighth fastener 18 comprises a buckle ring respectively corresponding to the first buckle 111, the second buckle 122, the third buckle 133, the fourth buckle 144, and the first upper buckle 301, the second upper buckle, the third upper buckle 303 and the fourth upper buckle of the upper back guard pad 30. The other end of each of the first fastener 11, the second fastener 12, the third fastener 13, the fourth fastener 14, the fifth fastener 15, the sixth fastener 16, the seventh fastener 17 and the eighth fastener 18 comprises a band ring, allowing an encircling band 80 to be passed through. The encircling band 80 comprises a fabric hook and loop fastener.

With reference to Fig. 1 and Fig. 3, the front guard pad 2000 comprises a lower front guard pad 50 and an upper front guard pad 60. The lower front guard pad 50 comprises a front end and a back end. The back end of the lower front guard pad 50 comprises a clasp 501. The upper front guard pad 60 comprises a front end. The front end of the upper front guard pad 60 comprises a clasp 601.

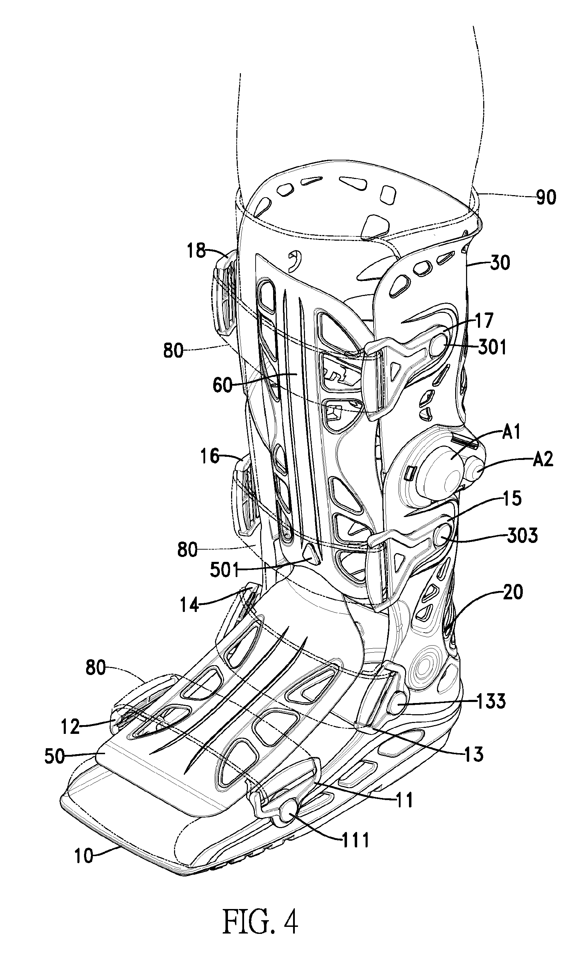

With reference to Fig. 1 and Fig. 4, a method for using the first embodiment comprises:
(1) putting an injured person's foot wrapped in a rehabilitation strap 90 on a surface 10A of the shoe body 10, and placing the back of the injured person's shank in the lower capacity space of the lower back guard pad 20 and the upper capacity space of the upper back guard pad 30.
(2) fastening the first fastener 11, the second fastener 12, the third fastener 13, and the fourth fastener 14 respectively with the first buckle 111, the second buckle 122, the third buckle 133 and the fourth buckle 144.
(3) placing the lower front guard pad 50 on an instep of the injured person's foot, such that the front end of the lower front guard pad 50 is corresponding to a tiptoe of the injured person's foot.
(4) passing the encircling band 80 through the band rings of the first fastener 11 and the second fastener 12, and the band rings of the third fastener 13 and the fourth fastener 14, separately.
(5) fixing the injured person's foot between the shoe body 10 and the lower front guard pad 50 by attaching the fabric hook and loop fastener of the encircling band 80.
(6) passing the third upper buckle 303, the fourth upper buckle, the first clasp 305 and the second clasp 306 through the first hole 203, the second hole 204, the first perforation 209 and the second perforation 210 of the lower back guard pad 20, respectively, allowing the first clasp 305 and the second clasp 306 to be clasped respectively with the first perforation 209 and the second perforation 210 of the lower back guard pad 20.
(7) fastening the buckle ring of the fifth fastener 15, the sixth fastener 16, the seventh fastener 17 and the eighth fastener 18 with the third upper buckle 303, the fourth upper buckle, the first upper buckle 301 and the second buckle of the upper back guard pad 30, respectively, allowing the lower back guard pad 20 to be fixed with the upper back guard pad 30.
(8) placing the upper front guard pad 60 on a front of the injured person's shank, and clasping the clasp 601 of the upper front guard pad 60 with the clasp 501 of the lower front guard pad 50.
(9) passing the encircling band 80 through the band rings of the fifth fastener 15 and the sixth fastener 16, and the band rings of the seventh fastener 17 and the eighth fastener 18, separately, allowing the injured person's shank to be fixed between the lower back guard pad 20, the upper back guard pad 30 and the upper front guard pad 20.

The first embodiment allows soft tissues as well as bones of the injured person's foot to be protected by fixing in a full-cover rehabilitation footwear, and avoids shifting of the soft tissues and bones to enhance recovery.

### The second embodiment

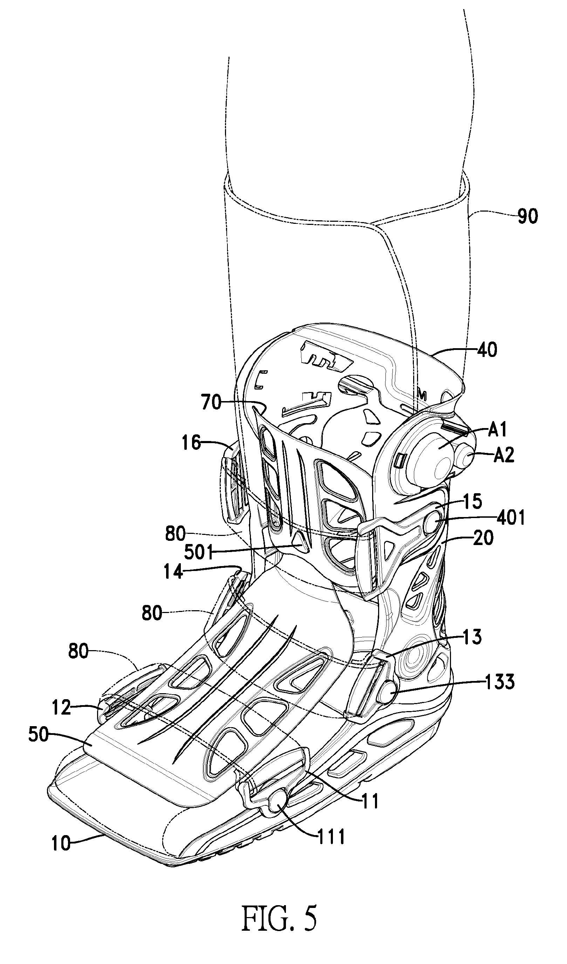

With reference to Fig. 1 and Fig. 5, a second embodiment of the rehabilitation footwear in accordance with the present invention comprises a shoe body 10, a back guard pad 1000, a front guard pad 2000, a first fastener 11, a second fastener 12, a third fastener 13, a fourth fastener 14, a fifth fastener 15, a sixth fastener 16, a first fill A and a second fill B.

The shoe body 10 comprises two lateral sides and a back end corresponding to bilateral sides and a heel of a human's foot, respectively. The two lateral sides and the back end of the shoe body 10 form a capacity space for accommodating the human's foot. The two lateral sides comprise a first buckle 111, a second buckle 122, a third buckle 133, and a fourth buckle 144. The back end of the shoe body 10 comprises a first concave groove 155 and a second concave groove 166.

The back guard pad 1000 comprises a lower back guard pad 20 and a middle back guard pad 40. The lower back guard pad 20 comprises three lateral sides connected with each other at nearly right angle, allowing to be formed a lower capacity space for accommodating the back of the human's shank. The lower capacity space comprises a lower end and an upper end. Two of the lateral sides of the lower back guard pad 20 opposite each other and corresponding to the lower end of the lower capacity space extend and form a first protrusion part 201 and a second protrusion part 202. The first protrusion part 201 and the second protrusion part 202 are respectively interlocked with the first concave groove 155 and the second concave groove 166 of the shoe body10, allowing the back guard pad 1000 to be fixed with the shoe body 10. The two opposite lateral sides of the lower back guard pad 20 comprise a first hole 203 and a second hole 204. The two lateral sides of the lower back guard pad 20 opposite with each other and corresponding to the upper end of the capacity space extend outwards and form a second small capacity space.

With reference to Fig. 1 and Fig. 2A, the second small capacity space corresponding to the two opposite lateral sides of the lower back guard pad 20 forms a third hole 205, a fourth hole 206, a fifth hole 207, a sixth hole 208, a first perforation 209, a second perforation 210, a third perforation 211 and a fourth perforation 212.

The middle back guard pad 40 is smaller than the upper back guard pad 30 and comprises three lateral sides connected with each other at nearly right angle, allowing to be formed a middle capacity space for accommodating the back of the human's shank. The middle capacity space comprises a lower end and an upper end.

The two lateral sides of the middle back guard pad 40 opposite each other and corresponding to the lower end of the middle capacity space comprise a first middle buckle 401 and a second middle buckle. The first middle buckle 401 and the second middle buckle pass through the first hole 203 and the second hole 204 of the lower back guard pad 20, respectively.

Two of the lateral sides of the middle back guard pad 40 opposite each other and corresponding to the upper end of the middle capacity space extend outwards and form protrusion structures. One of the protrusion structures is a second protrusion structure 40A. The second protrusion structure 40A comprises a surrounding wall having a second recess, and the first fill A is placed in the second recess of the second protrusion structure 40A. The second protrusion structure 40A is fitted into the small capacity space of the lower back guard pad 20. The other one of the protrusion structures is identical to the second protrusion structure 40A and positioned oppositely to the second protrusion structure 40A of the middle back guard pad 40. The other one of the protrusion structures comprises a surrounding wall having a recess formed threin, and the second fill B is placed in the recess of the other one of the protrusion structures. The second protrusion structure 40A and the second fill B are fit into the small capacity space of the two opposite lateral sides of the lower back guard pad 20. The first fill A comprises a first bump A1 and a second bump A2. The second fill B comprises a third bump B1 and a fourth bump. The first bump A1 passes through the third hole 205 of the lower back guard pad 20, and the second bump A2 passes through the fourth hole 206 of the lower back guard pad 20. The third bump B1 passes through the fifth hole 207 of the lower back guard pad 20, and the fourth bump passes through the sixth hole 208 of the lower back guard pad 20.

Two lateral sides of the surrounding wall of the second protrusion structure 40A comprise a third clasp 403 and a fourth clasp 404 formed toward outside of the recess. The third clasp 403 passes through and is clasped with the first perforation 209 of the lower back guard pad 20. The fourth clasp 404 passes through and is clasped with the second perforation 210 of the lower back guard pad 20, allowing the lower back guard pad 20 to be fixed with the middle back guard pad 40.

The first fastener 11, the second fastener 12, the third fastener 13, the fourth fastener 14, the fifth fastener 15, the sixth fastener 16, the seventh fastener 17 and the eighth fastener 18 each comprise two ends. One end of each of the first fastener 11, the second fastener 12, the third fastener 13, the fourth fastener 14, the fifth fastener 15, the sixth fastener 16, the seventh fastener 17 and the eighth fastener 18 comprises a buckle ring corresponding respectively to the first buckle 111, the second buckle 122, the third buckle 133, the fourth buckle 144, and the first middle buckle 401, the second middle buckle of the middle back guard pad 40. The other end of each of the first fastener 11, the second fastener 12, the third fastener 13, the fourth fastener 14, the fifth fastener 15, and the sixth fastener 16 comprises a band ring, allowing an encircling band 80 to be passed through. The encircling band 80 comprises a fabric hook and loop fastener.

With reference to Fig. 1 and Fig. 5, the front guard pad 2000 comprises a lower front guard pad 50 and a middle front guard pad 70. The lower front guard pad 50 comprises a front end and a back end. The back end of the lower front guard pad 50 comprises a clasp 501. The middle front guard pad 70 comprises a front end. The front end of the middle front guard pad 70 comprises a clasp 701.

A method for using the first embodiment comprises:
(1) putting an injured person's foot wrapped in the rehabilitation strap 90 on the surface 10A of the shoe body 10, and placing the back of the injured person's shank in the lower capacity space of the lower back guard pad 20 and the upper capacity space of the upper back guard pad 30.
(2) fastening the first fastener 11, the second fastener 12, the third fastener 13, and the fourth fastener 14 respectively with the first buckle 111, the second buckle 122, the third buckle 133 and the fourth buckle 144.
(3) placing the lower front guard pad 50 on an instep of the injured person's foot, such that the front end of the lower front guard pad 50 is corresponding to a tiptoe of the injured person's foot.
(4) passing the encircling band 80 through the band rings of the first fastener 11 and the second fastener 12, and the band rings of the third fastener 13 and the fourth fastener 14, separately.
(5) fixing the injured person's foot between the shoe body 10 and the lower front guard pad 50 by attaching the fabric hook and loop fastener of the encircling band 80.
(6) passing the first middle buckle 401, the second middle buckle, the third clasp 403 and the fourth clasp 404 through the first hole 203, the second hole 204, the first perforation 209 and the second perforation 210 of the lower back guard pad 20, respectively, allowing the third clasp 403 and the fourth clasp 404 to be clasped respectively with the first perforation 209 and the second perforation 210 of the lower back guard pad 20.
(7) fastening the buckle rings of the fifth fastener 15 and the sixth fastener 16 with the first middle buckle 401, the second middle buckle of the middle back guard pad 40, respectively, allowing the lower back guard pad 20 to be fixed with the middle back guard pad 40.
(8) placing the middle front guard pad 70 on a front of the injured person's shank, and clasping the clasp 701 of the middle front guard pad 70 with the clasp 501 of the lower front guard pad 50.
(9) passing the encircling band 80 through the band rings of the fifth fastener 15 and the sixth fastener 16, and the band rings of the seventh fastener 17 and the eighth fastener 18, separately, allowing the injured person's shank to be fixed between the lower back guard pad 20, the middle back guard pad 40 and the upper middle guard pad 70.

According to recovery of the injured person's foot, when ready for the next rehabilitation stage, the rehabilitation footwear of the fist embodiment can be replaced by the second embodiment. The rehabilitation footwear of the second embodiment allows the soft tissues as well as bones of the injured person's foot to be protected by fixing in a supporting-type rehabilitation footwear further for enhancing the rate of recovery.

### The third embodiment

With reference to Fig. 1, a third embodiment of the rehabilitation footwear in accordance with the present invention comprises a shoe body 10, frames, a first fastener 11, a second fastener 12, a third fastener 13, a fourth fastener 14, a fifth fastener 15 and a sixth fastener 16.

The shoe body 10 comprises two lateral sides and a back end corresponding to bilateral sides and a heel of a human's foot, respectively. The two lateral sides and the back end of the shoe body 10 form a capacity space for accommodating the human's foot. The two lateral sides comprise a first buckle 111, a second buckle 122, a third buckle 133, and a fourth buckle 144. The back end of the shoe body 10 comprises a first concave groove 155 and a second concave groove 166.

The frames comprise a first supporting frame 100 and a second supporting frame 110. Each of the first supporting frame 100 and the second supporting frame 110 comprises a lower end, a middle end, an upper end and two lateral sides.

The lower ends of the first supporting frame 100 and the second supporting frame 110 extend and respectively form a first protrusion part 1001 and a second protrusion part 1101. The first protrusion part 1001 of the first supporting frame 100 and the second protrusion part 1101 of the second supporting frame 110 are respectively interlocked with the first concave groove 155 and the second concave groove 166 of the shoe body10. The two lateral sides of the middle end of the first supporting frame 100 comprise a first band ring 1001A and a second band ring 1001B; the two lateral sides of the upper end of the first supporting frame 100 comprise a third band ring 1001C and a fourth band ring 1001D. The two lateral sides of the middle end of the second supporting frame 110 comprise a fifth band ring 1101A and a sixth band ring 1101B; the two lateral sides of the upper end of the second supporting frame 110 comprises a seventh band ring 1101C and an eighth band ring 1101D.

The first fastener 11, the second fastener 12, the third fastener 13, the fourth fastener 14, the fifth fastener 15 and the sixth fastener 16 each comprise two ends. One end of each of the first fastener 11, the second fastener 12, the third fastener 13, the fourth fastener 14, the fifth fastener 15 and the sixth fastener 16 comprises a buckle ring corresponding respectively to the first buckle 111, the second buckle 122, the third buckle 133, the fourth buckle 144, allowing an encircling band 80 to be passed through. The encircling band 80 comprises a fabric hook and loop fastener.

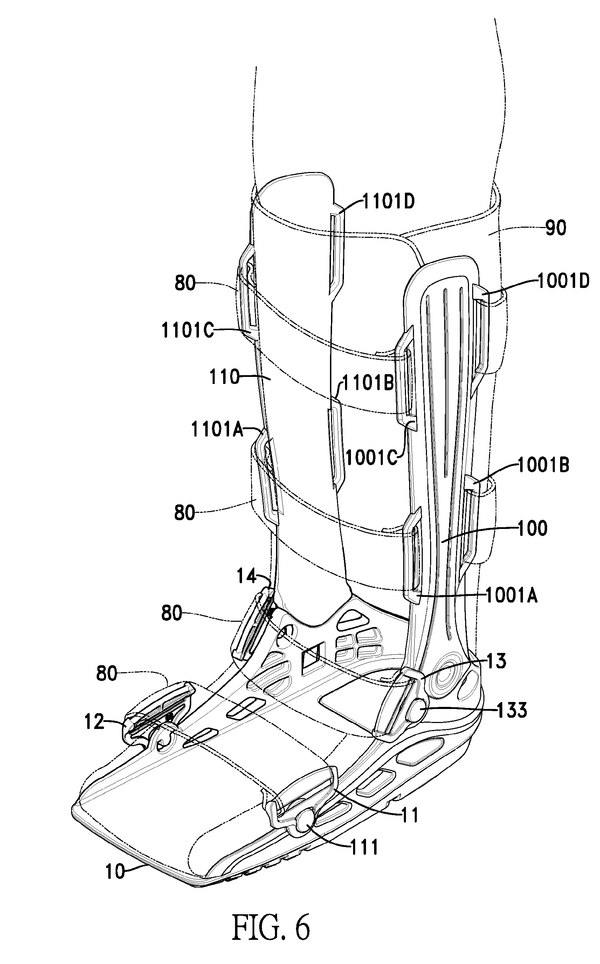

With reference to Fig. 1 and Fig. 6, a method for using the first embodiment comprises:
(1) wrapping an injured person's foot with the rehabilitation strap 90 and placing the injured person's foot on the surface 10A of the shoe body 10.
(2) fastening the first fastener 11, the second fastener 12, the third fastener 13, and the fourth fastener 14 respectively with the first buckle 111, the second buckle 122, the third buckle 133 and the fourth buckle 144.
(3) passing the encircling band 80 through the band rings of the first fastener 11 and the second fastener 12, and the band rings of the third fastener 13 and the fourth fastener 14, separately.
(4) fixing the injured person's foot with the shoe body 10 by attaching the fabric hook and loop fastener of the encircling band 80.
(5) passing the encircling band 80 through the first band ring 1001A of the first supporting frame 100 and the fifth band ring 1101A of the second supporting frame 110, separately; passing the encircling band 80 through the second band ring 1001B of the first supporting frame 100 and the sixth band ring 1101B of the second supporting frame 110, separately; passing the encircling band 80 through the third band ring 1001C of the first supporting frame 100 and the seventh band ring 1101C of the second supporting frame 110, separately; finally, passing the encircling band 80 through the fourth band ring 1001 D of the first supporting frame 100 and the eighth band ring 1101D of the second supporting frame 110, separately, allowing the injured person's shank to be fixed between the first supporting frame 100 and the second supporting frame 110.

According to recovery of the injured person's foot, when ready for next rehabilitation stage, the rehabilitation footwear of the second embodiment can be replaced by the third embodiment. The rehabilitation footwear of the second embodiment allows the soft tissues as well as bones of the injured person's foot to be protected by fixing in a frame-type rehabilitation footwear further for enhancing the rate of recovery.

## Claims

1. A rehabilitation footwear comprises:
a shoe body (10) comprising
two lateral sides corresponding to bilateral sides of a human's foot and comprising
multiple buckles (111, 122, 133, 144) positioned on the two lateral sides, and
two concave grooves (155, 166) positioned on the two lateral sides close to a heel of the shoe body (10);
multiple fasteners (11, 12, 13, 14) corresponding to the multiple buckles (111, 122, 133, 144) of the shoe body (10) and each fastener having two ends; one end of each of the multiple fasteners (11, 12, 13, 14) comprising a buckle ring fastened with one of the multiple buckles (111, 122, 133, 144) of the shoe body (10), and the other end of each of the multiple fasteners (11, 12, 13, 14) comprising a band ring;
an encircling band (80) passing through the band rings of the multiple fasteners (11, 12, 13, 14) positioned opposite each other;
a back guard pad (1000) comprising
a lower back guard pad (20) having two lateral sides, and each of the two lateral sides of the lower back guard pad (20) comprising
a lower end comprising a protrusion part (201, 202) interlocked with one of the two concave grooves (155, 166) of the shoe body (10);
multiple holes (203, 204, 205, 206, 207, 208), and
an upper end comprising multiple perforations (209, 210, 211, 212);
a middle back guard pad (40) having two lateral sides, and each of the two lateral sides of the middle back guard pad (40) comprising
a lower end comprising multiple buckles (401) passing through the multiple holes (203, 204) of the lower back guard pad (20) and fastened with the buckle rings of the multiple fasteners (15, 16);
an upper end comprising multiple clasps (403, 404) clasped with the multiple perforations (211, 212) of the lower back guard pad (20), and
a height;
an upper back guard pad (30) having two lateral sides, and each of the two lateral sides of the upper back guard pad (30) comprising
a lower end comprising multiple buckles (301, 303) passing through the multiple holes (203, 204) of the lower back guard pad (20) and fastened with the buckle rings of the multiple fasteners (15, 16, 17, 18);
an upper end comprising multiple clasps (305, 306) clasped with the multiple perforations (211, 212) of the lower back guard pad (20);
a height;
a front guard pad (1000) comprising
a lower front guard pad (50) having a back end and comprising a clasp (501);
a middle front guard pad (70) having
a length, and
a front end comprising a perforation (701) clasped with the clasp (501) of the lower front guard pad (50);
an upper front guard pad (60) having
a length, and
a front end comprising a perforation (601) clasped with the clasp (501) of the lower front guard pad (50);
two supporting frames (100, 110) and each of the two supporting frames (100, 110) comprising
a lower end having a protrusion part (1001, 1101) interlocked with the concave groove (155, 166) of the shoe body (10), and
two lateral sides.

2. The rehabilitation footwear as claimed in claim 1, wherein the upper end of the upper back guard pad (30) comprises at least one buckle (301) fastened with each of the buckle rings of the multiple fasteners (17, 18).

3. The rehabilitation footwear as claimed in claim 1, wherein the two lateral sides of each of the two supporting frames (100, 110) comprise multiple band rings (1001A, 1001B, 1001C, 100CD, 1101A, 1101B, 1101C, 1101D), wherein two of the multiple band rings (1001A, 1001B, 1001C, 100CD, 1101A, 1101B, 1101C, 1101D) are positioned opposite each other; and an encircling band 80 passes through the multiple band rings (1001A, 1001B, 1001C, 100CD, 1101A, 1101B, 1101C, 1101D) positioned corresponding to each other.

4. The rehabilitation footwear as claimed in claim 1, wherein the height of the upper back guard pad (30) is higher than the height of the middle back guard pad (40).

5. The rehabilitation footwear as claimed in claim 1, wherein the length of the upper front guard pad (60) is longer than the length of the middle front guard pad (70).

6. The rehabilitation footwear as claimed in claim 3, wherein the encircling band (80) comprises a fabric hook and loop fastener.
